# EUROPEAN PATENT SPECIFICATION

(11) **EP 3 974 011 B1**
(45) Date of publication and mention of the grant of the patent: **14.02.2024**
(21) Application number: 21190207.7
(22) Date of filing: 06.08.2021
(51) Int. Cl.: A61M 5/142

(54) **SELF-RETAINING DEVICE**
SELBSTHALTENDE VORRICHTUNG
DISPOSITIF À RETENUE AUTOMATIQUE

(30) Priority: 29.09.2020 JP 2020163569
(43) Date of publication of application: 30.03.2022
(73) Proprietor: TERUMO Kabushiki Kaisha, Tokyo 151-0072 (JP)
(72) Inventor: HATTORI, Tomohiro, Showa-cho, 409-3853 (JP); SAWAZAKI, Ryoichi, Showa-cho, 409-3853 (JP)
(74) Representative: Casalonga

(56) References cited:
- WO-A1-2017/122234
- US-A1- 2009 012 472
- US-A1- 2013 310 753
- US-A1- 2015 351 689
- US-A1- 2020 085 349

## Description

### BACKGROUND

### Technical Field

The present invention relates to a self-retaining device that is retained on a body surface of a living body.

### Related Art

JP 2013-63105 A discloses a medicine administration device that is retained on a body surface (self-retaining device) in order to administer liquid medicine into a body of a living body. The self-retaining device has a main body to which liquid medicine is administered, and an attachment sheet (attachment member) that is attached to the main body and is also attached to a body surface when the main body is retained on the body surface. In addition, a case of the self-retaining device can be deformed according to a curved shape of the body surface in order to reduce peeling of the main body off from the attachment member after a long period of retention.

However, as in JP 2013-63105 A, in a case of a structure in which the case is deformed, a problem of a complicating device, an increase in a manufacturing cost, or the like may arise. Furthermore, in addition to peeling between the main body and the attachment member, there may also be a case with this type of self-retaining device where an attachment member peels off from a body surface due to vibration (in a vertical direction) of the main body in daily life of a user.

For example, in a case where the main body is rising over the attachment member so that the attachment member can follow the body surface, a large torque load of vibration is applied to a boundary between a rising portion of the main body and a stuck portion of the main body. Therefore, the attachment member easily peels off from a periphery of a boundary of a rising portion of the main body and a stuck portion of the main body. Further prior art is constituted by US 2009/012472 A1 and US 2015/351689 A1.

The present invention relates to a technique of a self-retaining device as described above, and intends to provide a self-retaining device capable of stably retaining a main body on a body surface of a living body by favorably reducing peeling of an attachment member off from the body surface.

### SUMMARY

In order to achieve the above object, one aspect of the present invention is a self-retaining device including a main body and an attachment member that is sheet-shaped and configured to be attached to a body surface of a living body in order to retain the main body, in which the attachment member has a first section fixed to a back surface of the main body, a second section that faces the back surface of the main body at a position adjacent to the first section and is able to separate from the main body, and a periphery section that is a section adjacent to an outside of the first section and the second section and is configured to be attached to the body surface, the periphery section includes a lateral extension part parallel to a direction in which the first section and the second section are arranged, the lateral extension part has a boundary adjacent part overlapping an extension line of a linear boundary between the first section and the second section, a first section adjacent part adjacent to the boundary adjacent part and the first section, and a second section adjacent part adjacent to the boundary adjacent part and the second section, and a width of the boundary adjacent part is equal to or wider than a maximum width of the first section adjacent part and is wider than a maximum width of the second section adjacent part.

The above-described self-retaining device can stably retain a main body on a body surface of a living body by favorably reducing peeling of an attachment member off from the body surface.

### BRIEF DESCRIPTION OF DRAWINGS

FIG. 1 is a perspective view showing an overall configuration of a self-retaining device (medicine administration device) according to a first embodiment of the present invention;
FIG. 2 is an exploded perspective view showing a retention structure of a main body;
FIG. 3 is a plan view showing a main-body-side attachment sheet;
FIG. 4 is a plan view showing the main body retained on a body surface of a living body;
FIG. 5A is a cross-sectional side view taken along the line VA-VA in FIG. 4 showing the main body retained on the body surface; FIG. 5B is a partial perspective view showing rising and moving of the main body with respect to the main-body-side attachment sheet;
FIG. 6 is an exploded perspective view showing a retention structure according to a second embodiment of the present invention;
FIG. 7 is a plan view showing a main-body-side attachment sheet of a retention structure according to a third embodiment of the present invention; and
FIG. 8 is a plan view showing a main-body-side attachment sheet of a retention structure according to a fourth embodiment of the present invention.

### DETAILED DESCRIPTION

Hereinafter, preferred embodiments of the present invention will be described in detail with reference to the accompanying drawings.

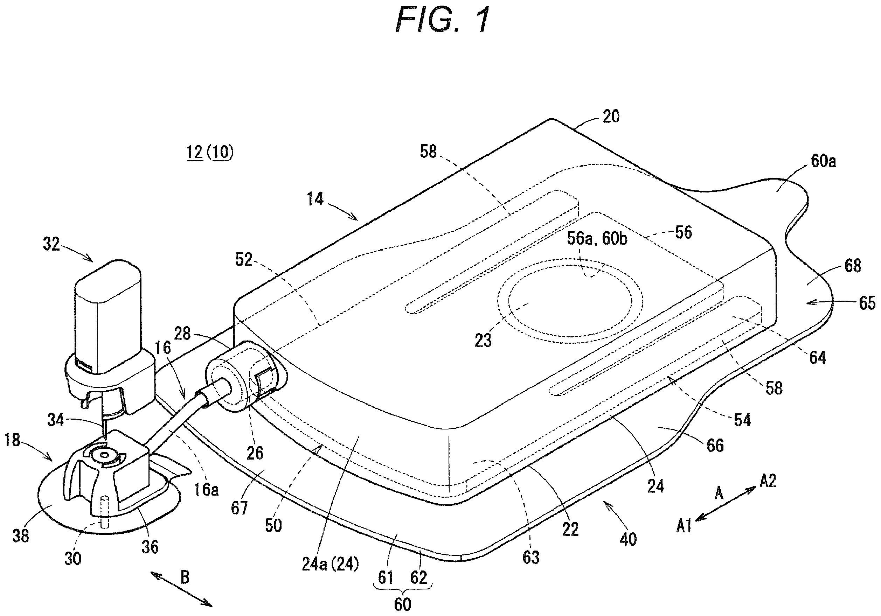

As a typical self-retaining device 10 according to a first embodiment of the present invention, a medicine administration device 12 shown in FIG. 1 will be described. The medicine administration device 12 is medical equipment retained on a body surface of a patient by a medical worker, or the like, and, when retained, automatically administers medicine into a body. It should be noted that the self-retaining device 10 according to the present invention is not limited to the medicine administration device 12, and may be applied to various devices retained on a body surface of a living body. Examples of the device include a component measurement device such as, for example, a blood glucose meter, a biosensor or other medical equipment, and a terminal such as an information processing device or a communication device, which is other than medical equipment.

The medicine administration device 12 includes a main body 14 that is a medicine supply source, a tube 16 connected to the main body 14, and an administration unit 18 connected to the tube 16 that administers liquid medicine supplied from the tube 16 to a patient. That is, in the medicine administration device 12, the main body 14 and the administration unit 18 are connected to each other via the tube 16, thereby ensuring a certain degree of mutual freedom. Therefore, the main body 14 and administration unit 18 of the medicine administration device 12 can be easily and accurately positioned on a desired position of the body surface when retained on the living body.

The main body 14 of the medicine administration device 12 has a container that stores medicine, a mechanism that delivers medicine from the container to the tube 16 at an appropriate timing, a control unit that controls operation of the mechanism, a power source for the mechanism or for the controller (both not illustrated), and the like. Examples of the medicine administered by the medicine administration device 12 include liquid medicinal drugs such as an antibody drug, an anticancer agent, a chemotherapeutic agent, an anesthetic, an antibiotic, insulin, a blood product, a nutrient, and the like. The main body 14 has a case 20 that accommodates the above-described configuration.

The case 20 has a large rectangular shape in plan view, and is configured as a substantially rectangular housing that is low in height in a thickness direction. The size of the case 20 is set within a range of, for example, about 30 mm to 150 mm long in a longitudinal direction, about 10 mm to 100 mm long in a transverse direction, and about 5 mm to 20 mm thick.

In the case 20, a back surface 22, which is a side to be retained on the body surface, is formed as a flat surface having a sufficient area. It should be noted that the back surface 22 may be formed slightly curved according to a shape of a body surface subjected to the retention. Four sides of the back surface 22 are continuous with four side surfaces 24 of the case 20 via R-shaped corners. In addition, four corners where the sides intersect with one another are formed into rounded corners. The case 20 constitutes a part of a retention structure 40 on which the main body 14 is retained.

An operation unit 23 for operating the main body 14 is provided on the back surface 22. The operation unit 23 according to the present embodiment is configured as a round push button. Power of the main body 14 is off at a time of product provision, and is configured such that once a user presses the operation unit 23, the control unit continues operation of the main body 14. A function of the operation unit 23 is not limited to turning power on or off, and the operation unit 23 may be applied to various configurations for operation of the self-retaining device 10. The main body 14 may include the operation unit 23 at a portion other than the back surface 22 of the case 20, and may not include the operation unit 23 in a case where operation is performed by external communication, or the like.

The case 20 is preferably configured elastically undeformable by a hard resin material. Although a resin material included in the case 20 is not particularly limited, thermoplastic resin such as, for example, polyacetal, polyamide, polyester, polycarbonate, polysulfone, polytetrafluoroethylene, polyphenylene sulfide, or polypropylene may be applied. It should be noted that the shape of the case 20 is not limited to a substantially rectangular shape, and various shapes such as a cubic shape, a hemispherical shape, or other three-dimensional shapes may be adopted.

A main-body-side connector 26 to be connected to the tube 16 is provided on one end in the longitudinal direction (arrow A directions) of the case 20. The main-body-side connector 26 projects short from a side surface 24a on one end side of the case 20.

The tube 16 of the medicine administration device 12 is a flexible tube having a flow passage 16a inside. Medicine can flow through the flow passage 16a that extends by a predetermined length. The length of the tube 16 is not particularly limited, but is set within a range of about 10 mm to 100 mm, for example. A tube-side connector 28 attachable to and removable from the main-body-side connector 26 is stuck to one end of the tube 16, and the administration unit 18 is fixed to another end of the tube 16. Mounted on the main-body-side connector 26, the tube-side connector 28 allows communication between a storage space of the container and the flow passage 16a of the tube 16.

The administration unit 18 of the medicine administration device 12 administers medicine to a patient via a catheter 30 inserted into the body (subcutaneously) from the body surface. In addition to the catheter 30, the administration unit 18 has a hub 36 that allows communication between the flow path in the catheter 30 and the flow passage 16a of the tube 16, and an administration-unit-side attachment sheet 38 that attaches the hub 36 on the body surface.

Before the catheter 30 is retained on the patient, the administration unit 18 is assembled into an insertion device 32, and an inner needle 34 of the insertion device 32 penetrates the catheter 30 (FIG. 1 shows the administration unit 18 and the insertion device 32 separately for ease of understanding of the present invention.). The user punctures the body with the multiple needle in which the catheter 30 and the inner needle 34 overlap with each other, releases the insertion device 32 from the administration unit 18 while keeping the multiple needle punctured, and pulls out the inner needle 34 from the catheter 30, thereby retaining the catheter 30 on the patient.

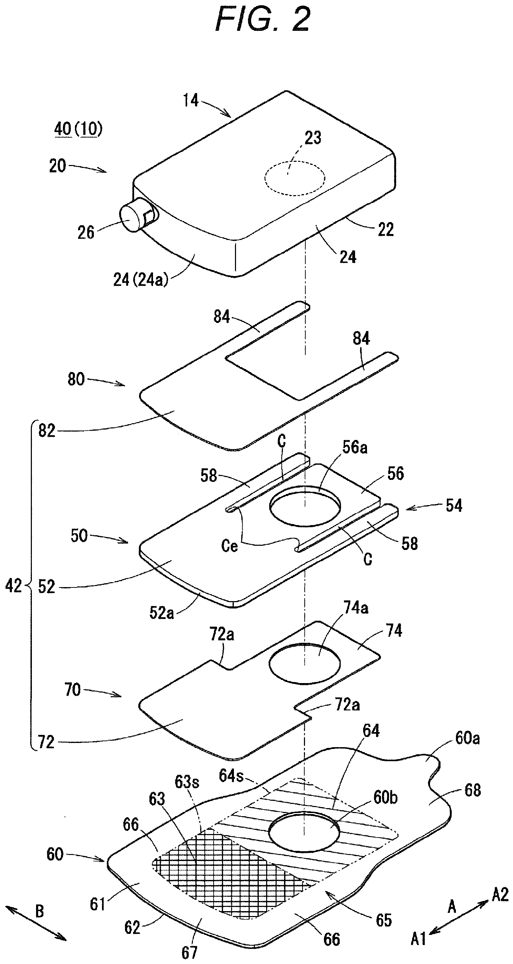

Next, the retention structure 40 in which the main body 14 of the medicine administration device 12 is retained on the body surface will be described with reference to FIG. 2. The retention structure 40 has the case 20 of the main body 14, a main-body-side attachment sheet 60 (attachment member) for attaching the main body 14 to a body surface of a living body, an intermediate member 50 laminated between the main body 14 and the main-body-side attachment sheet 60, a first sticking part 70 that sticks the main-body-side attachment sheet 60 and the intermediate member 50, and a second sticking part 80 that sticks the main body 14 and the intermediate member 50. In the retention structure 40, in a state where the main body 14 is retained on the body surface, the main-body-side attachment sheet 60 is fixed on the one end side (arrow A1 side) of the main body 14 via the intermediate member 50, while another end side (arrow A2 side) of the main body 14 can be separated from the main-body-side attachment sheet 60.

The intermediate member 50 is formed in an elastically deformable plate shape. The intermediate member 50 supports separation (rising) of the main body 14 while keeping the main body 14 and the main-body-side attachment sheet 60 stuck to each other, thereby reinforcing attachment of the main-body-side attachment sheet 60 to the main body 14 and attachment of the main-body-side attachment sheet 60 to the body surface.

Specifically, the intermediate member 50 is formed sufficiently thinner than thickness of the case 20 and is configured to be elastically curved. The thickness of the intermediate member 50 is not particularly limited, but is preferably set within a range of about 0.1 mm to 3 mm, for example. For example, a degree of elasticity of the intermediate member 50 is set to be larger than a degree of elasticity of the body surface. It should be noted that the degree of elasticity of the intermediate member 50 may be set to substantially equal to or slightly smaller than elasticity of the body surface.

The intermediate member 50 is formed in a substantially rectangular shape corresponding to a shape of the back surface 22 of the case 20 in plan view. The plan shape of the intermediate member 50 substantially coincides with a shape of the back surface 22 of the main body 14. That is, the intermediate member 50 is stuck exclusively to the back surface 22, instead of being attached to an R-shaped corner provided between the back surface 22 and side surface 24 of the case 20. It should be noted that the intermediate member 50 may be formed slightly smaller or slightly larger than a shape of the back surface 22.

The intermediate member 50 has a base part 52 constituting substantially a half of one end side (arrow A1 side) in the longitudinal direction of the intermediate member 50 and a plurality of elastic pieces 54 that constitutes substantially a half of another end side (arrow A2 side) in the longitudinal direction of the intermediate member 50. A ratio of a length of the base part 52 to a length of the plurality of elastic pieces 54 in the longitudinal direction of the intermediate member 50 is not particularly limited. For example, the length of the base part 52 in the longitudinal direction may be set within a range of between 30% and 70% of the length of the intermediate member 50 in the longitudinal direction, and a length of the plurality of elastic pieces 54 in the longitudinal direction may be set as appropriate according to a ratio of the base part 52 to the intermediate member 50.

The base part 52 is attached to substantially a half of the case 20 on the arrow A1 side, and is attached to substantially a half of the main-body-side attachment sheet 60 on the arrow A1 side. Therefore, as shown in FIG. 2, the first sticking part 70 is attached to a lower surface of the base part 52, and a second sticking part 80 is attached to an upper surface of the base part 52. The base part 52 is disposed on the arrow A1 side of the main body 14 near the main-body-side connector 26 to strengthen a stuck state of a periphery of a portion of the main body 14 where medicine flows out. An end side 52a of the base part 52 on the arrow A1 side is formed in an arc shape in accordance with a shape of the side surface 24a of the case 20.

The plurality of elastic pieces 54 includes a central piece 56 provided at a center of the intermediate member 50 in the transverse direction, and a pair of arm pieces 58 provided on both sides of the central piece 56 in the transverse direction. The central piece 56 and each of the pair of arm pieces 58 continue flush with the base part 52 and extend in parallel to each other from the base part 52. A clearance C is provided between the central piece 56 and each of the pair of arm pieces 58 to separate the pieces from each other.

The central piece 56 is formed in a rectangular shape extending from the base part 52 toward the direction of the arrow A2. The central piece 56 is attached to the main-body-side attachment sheet 60 and constitutes a first extending part that is not attached to the back surface 22 of the case 20. Therefore, the first sticking part 70 can be attached to a lower surface of the central piece 56, while the second sticking part 80 cannot be attached on an upper surface of the central piece 56. The central piece 56 is provided with an intermediate-member-side exposure hole 56a through which the operation unit 23 is exposed.

The pair of arm pieces 58 is formed in an elongated plate shape extending from the base part 52 toward the direction of the arrow A2 by the same length as the central piece 56. Each of the arm pieces 58 is not attached to the main-body-side attachment sheet 60 and constitutes a second extending part that is attached to the back surface 22 of the case 20. Therefore, the first sticking part 70 cannot be attached to a lower surface of each of the arm pieces 58, while the second sticking part 80 can be attached on an upper surface of each of the arm pieces 58.

A pair of clearances C provided between the central piece 56 and the pair of arm pieces 58 extends from the base part 52 toward the direction of the arrow A2. An arc-shaped edge Ce in contact with the base part 52 is formed at one end of each of the clearances C (a root where the central piece 56 and the pair of arm pieces 58 are connected to the base part 52).

Examples of a constituent material of the intermediate member 50 include, for example, polymer materials such as polyethylene, polypropylene, polyethylene terephthalate, polyvinyl chloride, polystyrene, ABS resin, acrylic, polyamide, polyester, and fluororesin, and mixtures thereof.

The first sticking part 70 is double-sided tape having an adhesive layer on a lower surface and upper surface thereof. The first sticking part 70 has a lower base part attachment region 72 attached to an entire lower surface of the base part 52 and a lower elastic piece attachment region 74 continuous with the lower base part attachment region 72 and attached to an entire lower surface of the central piece 56. The lower elastic piece attachment region 74 has an adhesive-member-side exposure hole 74a at a position corresponding to the intermediate-member-side exposure hole 56a.

The second sticking part 80 is double-sided tape having an adhesive layer on a lower surface and upper surface thereof. The second sticking part 80 has an upper base part attachment region 82 attached to the base part 52 and a pair of upper elastic piece attachment region 84 continuous with the upper base part attachment region 82 and attached to the pair of arm pieces 58. It should be noted that the first sticking part 70 and the second sticking part 80 may be formed by directly applying an adhesive to the lower surface or upper surface of the intermediate member 50.

In the retention structure 40 described above, the main body 14, the second sticking part 80, the intermediate member 50, the first sticking part 70, and the main-body-side attachment sheet 60 are laminated in this order from the upper side to the lower side. However, to the main-body-side attachment sheet 60, the base part 52 and central piece 56 of the intermediate member 50 are attached, while the pair of arm pieces 58 is not attached. Further, to the case 20 of the main body 14, the base part 52 and the pair of arm pieces 58 are attached, while the central piece 56 is not attached. The upper base part attachment region 82 of the second sticking part 80, the base part 52 of the intermediate member 50, and the lower base part attachment region 72 of the first sticking part 70 are laminated between the main body 14 and the main-body-side attachment sheet 60 and constitute a sticking part 42 that defines a first section 63 of the main-body-side attachment sheet 60. The first section 63 will be described later. Meanwhile, the upper elastic piece attachment region 84 of the second sticking part 80, the elastic pieces 54 of the intermediate member 50, the lower elastic piece attachment region 74 of the first sticking part 70 are laminated between the main body 14 and the main-body-side attachment sheet 60 and defines a second section 64 of the main-body-side attachment sheet 60. The second section 64 will be described later.

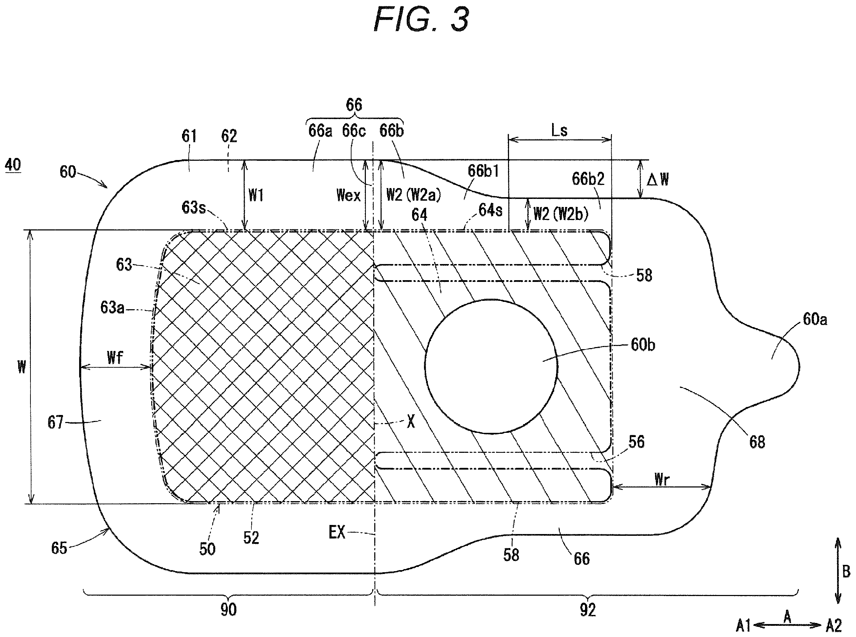

As shown in FIGS. 2 and 3, the main-body-side attachment sheet 60 is formed in a substantially rectangular shape in plan view, corresponding to the shape of the case 20, and has flexibility capable of following the body surface. In the main-body-side attachment sheet 60, an end side on the arrow A1 side corresponding to the main-body-side connector 26 of the main body 14 is formed in an arc shape corresponding to the end side 52a of the base part 52 (side surface 24a of the case 20). In the main-body-side attachment sheet 60, a projection piece 60a is formed on an end side of the direction of the arrow A2. The projection piece 60a constitutes a portion pinched by the user when the main-body-side attachment sheet 60 is attached or detached.

The main-body-side attachment sheet 60 is formed by laminating a base material 61 and an adhesive layer 62. An appropriate material is preferably applied to the base material 61 in consideration of stretchability, followability, breathability, and the like. Examples of the material of the base material 61 include, for example, polyethylene, polyester, polyurethane, nylon, polyolefin, cotton fabric, woven fabric, and non-woven fabric. In addition, with arbitrary processing, the base material 61 may be configured to have a function such as water repellency, hydrophilicity, irregularities, an air hole, moisture permeability, and flexibility.

The adhesive layer 62 is formed on an entire lower surface of the base material 61. The entire lower surface faces the body surface. As a material of the adhesive layer 62, for example, an acrylic-based adhesive, a silicone adhesive, a urethane-based adhesive, a synthetic rubber-based adhesive, or the like is applied. The lower surface of the main-body-side attachment sheet 60 may have not only the adhesive layer 62 provided on the entire surface but also a non-adhesive region where the adhesive layer 62 is not applied to a part of the lower surface (for example, the projection piece 60a).

The main-body-side attachment sheet 60 has the first section 63 (cross-hatched area in FIGS. 2 and 3) at a position overlapping with the base part 52, and has the second section 64 (hatched area in FIGS. 2 and 3) at a position adjacent to the first section 63 and overlapping with the plurality of elastic pieces 54 of the intermediate member 50. In practice, the first section 63 and the second section 64 are not visible on the base material 61. However, in FIGS. 2 and 3, lines 63s and 64s defining the respective sections are indicated by two-dot chain lines for convenience.

That is, the first section 63 is a section that indirectly fixes the flat back surface 22 of the facing main body 14 via the base part 52. The second section 64 is a section that allows the back surface 22 of the main body 14 facing each other to rise over the main-body-side attachment sheet 60 via the central piece 56 and the pair of arm pieces 58. This is because the second section 64 is stuck to the central piece 56 not stuck to the main body 14, and is not stuck to the pair of arm pieces 58 stuck to the main body 14.

Specifically, the first section 63 is a portion to which the lower base part attachment region 72 of the first sticking part 70 is attached, and substantially coincides with a shape of the base part 52 of the intermediate member 50. The first section 63 is positioned on the arrow A1 side from vicinity of a central portion of the main-body-side attachment sheet 60 in the longitudinal direction (arrow A directions). The first section 63 is formed in a rectangular shape slightly longer in the transverse direction (arrow B directions) of the main-body-side attachment sheet 60. An end side 63a of the first section 63 on the arrow A1 side is formed in an arc shape in accordance with the end side 52a of the base part 52 of the intermediate member 50 (the side surface 24a of the case 20) .

The second section 64 includes a portion to which the lower elastic piece attachment region 74 of the first sticking part 70 is attached and a portion facing the pair of arm pieces 58 and the pair of clearances C of the intermediate member 50. The second section 64 is positioned on the arrow A2 side from vicinity of a central portion of the main-body-side attachment sheet 60 in the longitudinal direction. The second section 64 is formed in a rectangular shape slightly longer in the transverse direction of the main-body-side attachment sheet 60. The second section 64 is provided with an attachment-member-side exposure hole 60b that penetrates the main-body-side attachment sheet 60 in the thickness direction and exposes the operation unit 23 of the main body 14.

A length of the second section 64 along the direction of arrow A is set to be slightly longer than (or substantially equal to) a length of the first section 63 along the arrow A directions. Meanwhile, a length of the second section 64 along the arrow B directions and a length of the first section 63 along the arrow B directions coincide with each other. A length W of the first section 63 and second section 64 along the arrow B directions can be set within a range of, for example, about 18 mm to 45 mm, and is set to 33 mm in the present embodiment.

Between the first section 63 and the second section 64, a boundary X for the sections is provided. The boundary X extends linearly along the arrow B directions of the main-body-side attachment sheet 60. That is, the boundary X is a straight line extending in a direction orthogonal to an arrangement direction of the first section 63 and the second section 64. The boundary X is positioned in vicinity of the attachment-member-side exposure hole 60b on the arrow A1 side of the attachment-member-side exposure hole 60b. The boundary X overlaps with a pair of sides 72a (refer to FIG. 2) on the arrow A2 side of the lower base part attachment region 72 of the first sticking part 70, and, when the medicine administration device 12 is retained, exactly overlaps with the boundary between the base part 52 and elastic pieces 54 of the intermediate member 50.

Further, the main-body-side attachment sheet 60 has a periphery section 65 that projects outward of the first section 63 and the second section 64 and is attached to the body surface. The periphery section 65 is a portion that does not face the back surface 22 of the main body 14 and is attached exclusively to the body surface by exposing the base material 61 of the main-body-side attachment sheet 60. That is, due to the periphery section 65, the main-body-side attachment sheet 60 is formed wider in the longitudinal direction and transverse direction than the back surface 22 of the main body 14.

The periphery section 65 has a pair of lateral extension parts 66 on both sides of the main-body-side attachment sheet 60 in the transverse direction. The pair of lateral extension parts 66 extends in parallel to a direction in which the first section 63 and the second section 64 are arranged (arrow A directions: longitudinal direction of the main-body-side attachment sheet 60). The periphery section 65 has a one-end extension part 67 extending in the arrow B directions at the end side on the arrow A1 side of the main-body-side attachment sheet 60 and an another-end extension part 68 extending in the arrow B directions at the end side on the arrow A2 side of the main-body-side attachment sheet 60.

The pair of lateral extension parts 66 has a first section adjacent part 66a adjacent to the first section 63, a second section adjacent part 66b adjacent to the second section 64, and a boundary adjacent part 66c that overlaps with an extension line EX obtained by extending the boundary X. That is, the first section adjacent part 66a is adjacent to the arrow A1 side of the boundary adjacent part 66c (side close to the first section 63), and the second section adjacent part 66b is adjacent to the arrow A2 side (side close to the second section 64) of the boundary adjacent part 66c.

The first section adjacent part 66a is a portion where a pair of outer edges of the main-body-side attachment sheet 60 linearly extends in parallel from the extension line EX toward the direction of the arrow A1. Therefore, a width W1 of the first section adjacent part 66a is constant in the arrow A directions, and thus a maximum width of the first section adjacent part 66a is the width W1. The width W1 is a dimension in a direction parallel to the extension line EX of the first section adjacent part 66a. The width W1 of the first section adjacent part 66a is set within a range of, for example, about 8.5 mm to 14 mm, and is set to 10.5 mm in the present embodiment. An end of the first section adjacent part 66a on the arrow A1 side is continuous with the one-end extension part 67.

The second section adjacent part 66b is a portion where a pair of outer edges of the main-body-side attachment sheet 60 changes inward from the extension line EX toward the direction of the arrow A2. Therefore, a width W2 of the second section adjacent part 66b gradually narrows toward the direction of the arrow A2. The width W2 is a dimension in a direction parallel to the extension line EX of the second section adjacent part 66b. Specifically, the second section adjacent part 66b has a width changing part 66b1 having an outer edge that gradually narrows from the extension line EX toward the direction of the arrow A2, and a straight part 66b2 continuous with the arrow A2 side of the width changing part 66b1 and linearly extending along the side of the arrow A2.

The arrow A1 side of the width changing part 66b1 has an outer edge farthest away from the second section 64 in the second section adjacent part 66b. Therefore, the width W2 of the second section adjacent part 66b has a width W2a (maximum width) at a position continuous with the boundary adjacent part 66c, and gradually narrows toward the direction of the arrow A2 from the position. However, the width W2a is slightly narrower than a width Wex of the boundary adjacent part 66c, which will be described later. Therefore, the width W2 of the second section adjacent part 66b is always formed narrower than the width Wex of the boundary adjacent part 66c. The width changing part 66b1 is smoothly continuous with the straight part 66b2 on the arrow A2 side.

The straight part 66b2 extends from the width changing part 66bl toward the direction of the arrow A2 in the second section adjacent part 66b, and is continuous with the another-end extension part 68 at an end on the arrow A2 side. A length Ls of the straight part 66b2 in the direction of the arrow A is shorter than the length of the width changing part 66b1 in the arrow A directions. The length Ls of the straight part 66b2 in the arrow A directions is set within a range of 1 mm to 15 mm, for example.

The straight part 66b2 extends with a constant width W2b by a pair of outer edges of the main-body-side attachment sheet 60 being in parallel along the arrow A directions. The straight part 66b2 corresponds to a narrowest portion in the lateral extension parts 66. The width W2b of the straight part 66b2 may be set within a range of, for example, about 5 mm to 7 mm, and is set to 6 mm in the present embodiment.

The boundary adjacent part 66c refers to an area on the extension line EX of the lateral extension parts 66 and about a range of 1 mm from the extension line EX toward the direction of the arrow A1 and toward the direction of the arrow A2. The boundary adjacent part 66c is provided at a substantially central portion in the longitudinal direction of the main-body-side attachment sheet 60. It should be noted that the position of the boundary adjacent part 66c is not limited to a substantially central portion in the longitudinal direction, and may be positioned in a middle of three regions obtained by dividing the main-body-side attachment sheet 60 into three equal parts along the longitudinal direction.

The width Wex of the boundary adjacent part 66c is a dimension in a direction parallel to the extension line EX of the boundary adjacent part 66c. The outer edge of the main-body-side attachment sheet 60 linearly extends from the first section adjacent part 66a to the boundary adjacent part 66c. Therefore, the width Wex of the boundary adjacent part 66c coincides with the width W1 of the first section adjacent part 66a (Wex = W1). That is, the width Wex of the boundary adjacent part 66c is equal to or wider than the maximum width of the first section adjacent part 66a. The width Wex of the boundary adjacent part 66c is set to be wider than the width W2 of the second section adjacent part 66b (Wex > W2).

In other words, the lateral extension parts 66 extend with a constant width W1 (= Wex) from the end on the arrow A1 side toward the direction of the arrow A2 to the extension line EX, gradually narrow from the extension line EX, and again extend with a constant width W2b in the straight part 66b2.

In this case, the width Wex of the boundary adjacent part 66c is within a range of about 8.5 mm to 14 mm, and as described above, the width W2b of the straight part 66b2 of the second section adjacent part 66b is within a range of about 5 mm to 7 mm. In other words, the width Wex of the boundary adjacent part 66c is set within a range of 1.4 times to 3.5 times the width W2b of the straight part 66b2. As a result, the lateral extension parts 66 are attached in a sufficiently wide area in vicinity of the extension line EX, and force to maintain attachment to the body surface is increased. If the width Wex is narrower than 1.4 times the width W2b, there is a possibility that sufficient force to maintain attachment cannot be obtained. Meanwhile, in a case where the width Wex is wider than 3.5 times the width W2b, the main-body-side attachment sheet 60 is too large. Therefore, there is a possibility that the main-body-side attachment sheet 60 interferes with movement of the living body (curve of an abdomen or the like), or that the main-body-side attachment sheet 60 is easily peeled off due to a wrinkle generated at a time of curving.

In other words, a difference ΔW obtained by subtracting the width W2b of the straight part 66b2 from the width Wex of the boundary adjacent part 66c is set within a range of 2.5 mm to 10 mm. Furthermore, because the width W of the first section 63 along an extending direction of the boundary X (arrow B directions) is within a range of about 18 mm to 45 mm, it can be said that the width Wex of the boundary adjacent part 66c is set within a range of about 1/5 to 4/5 of the width W of the first section 63.

The one-end extension part 67 of the periphery section 65 is provided on the arrow A1 side of the first section 63, and extends in an arc shape with a constant width Wf along the arrow B directions. The width Wf of the one-end extension part 67 is a dimension in a direction (arrow A directions) orthogonal to the boundary X. Both ends of the one-end extension part 67 in the arrow B directions are continuous with each of the first section adjacent part 66a of the pair of lateral extension parts 66 via the R-shaped corners. The width Wf of the one-end extension part 67 coincides with the width W1 of the first section adjacent part 66a of each of the lateral extension parts 66. That is, the width Wf of the one-end extension part 67 is set within a range of about 8.5 mm to 14 mm, and is set to 10.5 mm in the present embodiment.

The another-end extension part 68 of the periphery section 65 is provided on the arrow A2 side of the second section 64, and both ends in the arrow B directions are continuous with the straight parts 66b2 of the second section adjacent parts 66b of the pair of lateral extension parts 66 via the R-shaped corners. The width Wr of the another-end extension part 68 is formed to be significantly wider than the width Wf of the one-end extension part 67. That is, the arrow A2 side of the main-body-side attachment sheet 60 allows the main body 14 to rise with the boundary X and the extension line EX as the base points, while has a wide area to constitute a portion that is configured to be attached to the body surface and follows the body surface.

For example, in a case where, of the main-body-side attachment sheet 60, a side close to the first section 63 including the periphery section 65 is defined as a first region 90 and a side close to the second section 64 including the periphery section 65 is defined as a second region 92 with the boundary X and the extension line EX as the base points, an area of the second region 92 is set to 1/4 or more of an area of the first region 90. As an example, in a case where the area of the first region 90 is about 2400 mm², the area of the second region 92 is set to 600 mm² or more. Accordingly, even if the main body 14 is configured to rise over the body surface, the second region 92 can firmly stick the main-body-side attachment sheet 60 to the body surface.

It should be noted that a shape of the second region 92 (another-end extension part 68) is not limited to the shape shown in FIG. 3, and may be a triangle or a semicircle having an R-shaped corner. In this case, the pair of lateral extension parts 66 continuous with the another-end extension part 68 may not include the straight part 66b2.

The self-retaining device 10 (medicine administration device 12) according to the present embodiment is basically configured as described above, and operation thereof will be described below.

To the user, the medicine administration device 12 is provided with the administration unit 18 and the insertion device 32 assembled, while with the tube 16, which is connected to the administration unit 18, and the main body 14 separated from each other. Then, for example, when retaining the medicine administration device 12, the user sequentially performs administration unit retention step of retaining the administration unit 18, a connection step of connecting the tube 16 coupled to the administration unit 18 to the main body 14, and a main body retention step of retaining the main body 14.

By the above-described retention work, the main body 14 of the medicine administration device 12 is stuck on the body surface via the retention structure 40. At the time of retention, the main body 14 is activated in response to the operation unit 23 being turned on. At this time, the operation unit 23 is exposed via the attachment-member-side exposure hole 60b and the intermediate-member-side exposure hole 56a, and therefore the user can easily operate the operation unit 23. Even after the operation unit 23 is left unoperated and the main-body-side attachment sheet 60 is attached to the body surface, the operation unit 23 can be exposed by curving the central piece 56 without peeling the main-body-side attachment sheet 60 off from the body surface.

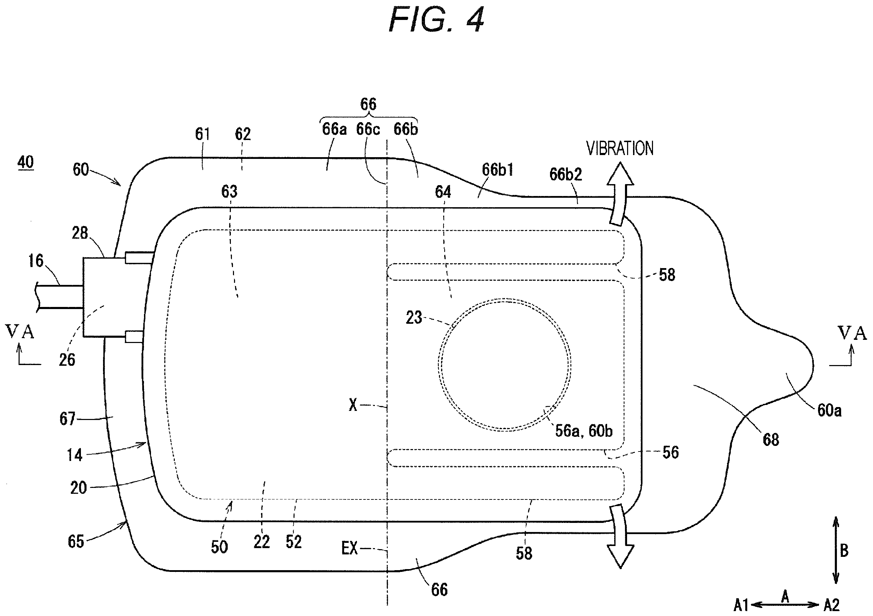

As shown in FIG. 4, in the main-body-side attachment sheet 60, the periphery section 65 is exposed around the main body 14 that is retained. By being attached to the body surface, the periphery section 65 reduces peeling of the main-body-side attachment sheet 60 even if force is applied from the main body 14 to the main-body-side attachment sheet 60.

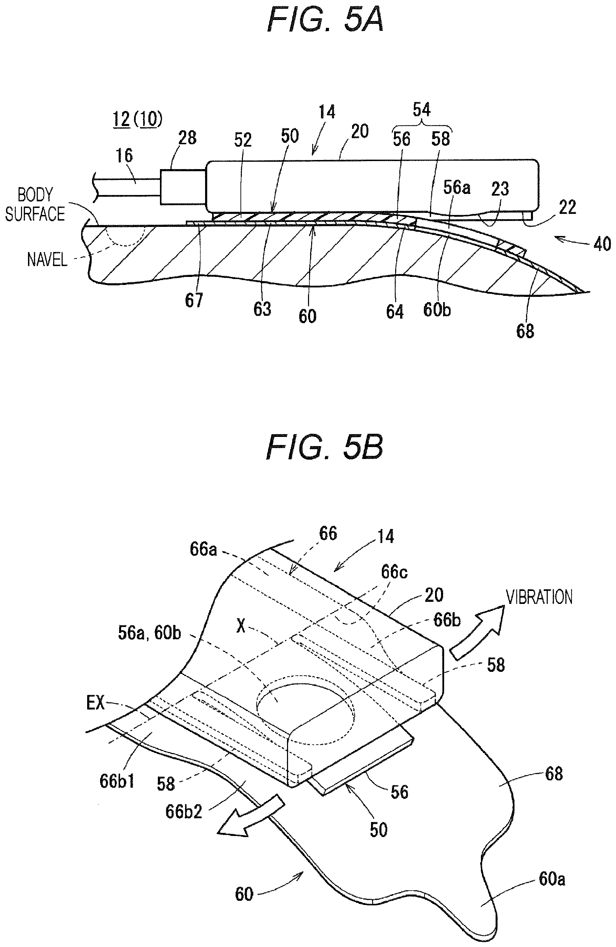

As shown in FIGS. 5A and 5B, for example, on an abdomen, the main body 14 of the medicine administration device 12 is retained such that a side close to the tube-side connector 28 (one end side: arrow A1 side) faces a center (navel) of the abdomen. At this time, the main-body-side attachment sheet 60 is attached onto skin such that the longitudinal direction of the main-body-side attachment sheet 60 is aligned along curved portions from the navel to sides of the patient. The one end side of the main body 14 is firmly stuck to the main-body-side attachment sheet 60 via the base part 52 of the intermediate member 50.

The central piece 56 of the another end side of the intermediate member 50 can be separated from the main body 14 when the medicine administration device 12 is retained, and is disposed close to the body surface following a curve of the body surface (main-body-side attachment sheet 60). Meanwhile, the pair of arm pieces 58 of the intermediate member 50 can rise over the main-body-side attachment sheet 60 while keeping stuck to the back surface 22 of the case 20 without following the body surface. In the retention structure 40 retained in this manner, peeling can be avoided by the intermediate member 50 elastically deforming, even if external force, which peels the main body 14 off from the body surface, is applied to the main body 14 due to contact in daily life, unexpected contact, or the like.

After being retained, the medicine administration device 12 automatically administers medicine to the patient under control of the control unit. Period of retaining the medicine administration device 12 (medicine administration period) is set to a range of, for example, about one week to four weeks.

If the patient walks or the like with the medicine administration device 12 retained, the arrow A2 side of the main body 14 vibrates in the vertical direction (refer to the white arrows in FIGS. 4 and 5B) around the first section 63 of the main-body-side attachment sheet 60 that adheres via the base part 52. Torque load caused by the vibration induces peeling of the main-body-side attachment sheet 60 from the body surface at the extension line EX of the boundary X. That is, the arrow A2 side of the main body 14 serves as a point of effort of the torque load at a time of vibration, and the first section 63 of the main-body-side attachment sheet 60 serves as a fulcrum of the torque load, by which a point of application of the torque load is applied to periphery of the boundary X of the main-body-side attachment sheet 60.

Here, in the periphery section 65 of the main-body-side attachment sheet 60, the width Wex of the boundary adjacent part 66c is wider than the width W2 (width W2b of the straight parts 66b2) of the second section adjacent part 66b on the arrow A2 side of the boundary X That is, the boundary adjacent part 66c is attached to a wide area of the body surface along the arrow B directions of the main-body-side attachment sheet 60, by which force to maintain attachment to the body surface is increased. Therefore, even if torque load due to vibration is applied to the boundary adjacent part 66c, the main-body-side attachment sheet 60 can stably keep attached to the body surface.

In addition, the first section adjacent part 66a on the arrow A1 side and the boundary adjacent part 66c are continuous with each other. The first section adjacent part 66a has the width W1 that is the same as the width Wex of the boundary adjacent part 66c. The one-end extension part 67 and the first section adjacent part 66a are continuous with each other. The one-end extension part 67 has the width Wf that is the same as the width W1 of the first section adjacent part 66a. That is, the main-body-side attachment sheet 60 is attached to a wide area of the body surface on the arrow A1 side of the boundary X and the extension line EX. As a result, in the main-body-side attachment sheet 60, followability to a wrinkle generated in a lateral direction of the abdomen is improved, and force to maintain attachment to the body surface is increased at an entire portion subjected to moving. As a result, the main-body-side attachment sheet 60 is more difficult to peel off from the body surface.

It should be noted that the present invention is not limited to the above embodiments, and various modifications can be made in accordance with the gist of the invention. For example, the retention structure 40 is not limited to the configuration in which the pair of lateral extension parts 66 of the main-body-side attachment sheet 60 is provided on both sides in the arrow B directions, and a lateral extension part 66 may be provided on one side (a side positioned on the upper side of a direction of gravity when attached to the body surface) of the arrow B directions.

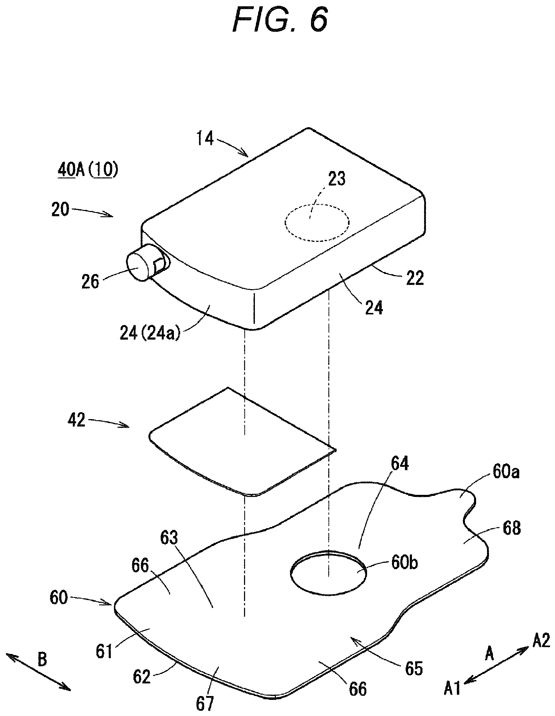

Further, for example, as in a second embodiment shown in FIG. 6, a retention structure 40A may have a configuration in which a main body 14 and main-body-side attachment sheet 60 are directly fixed (adhered) without interposing of an intermediate member 50. It should be noted that, in the following description, components having substantially the same functional configuration as the above-described embodiments are provided with the same reference signs, so that detailed description thereof will be omitted.

The retention structure 40A has the main body 14, the main-body-side attachment sheet 60, and a sticking part 42 that sticks both members, the main body 14 and the main-body-side attachment sheet 60. The sticking part 42 is attached to an arrow A1 side of the main body 14 and a first section 63 of the main-body-side attachment sheet 60. Therefore, a second section 64 of the main-body-side attachment sheet 60 and a back surface 22 of the main body 14 are not stuck to each other, and an arrow A2 side of the main body 14 can rise over the main-body-side attachment sheet 60. In the retention structure 40A also, a width Wex of a boundary adjacent part 66c of the main-body-side attachment sheet 60 is wider than a width W2 of a second section adjacent part 66b. Therefore, even if the main body 14 moves, the main-body-side attachment sheet 60 can stably keep attached to a body surface.

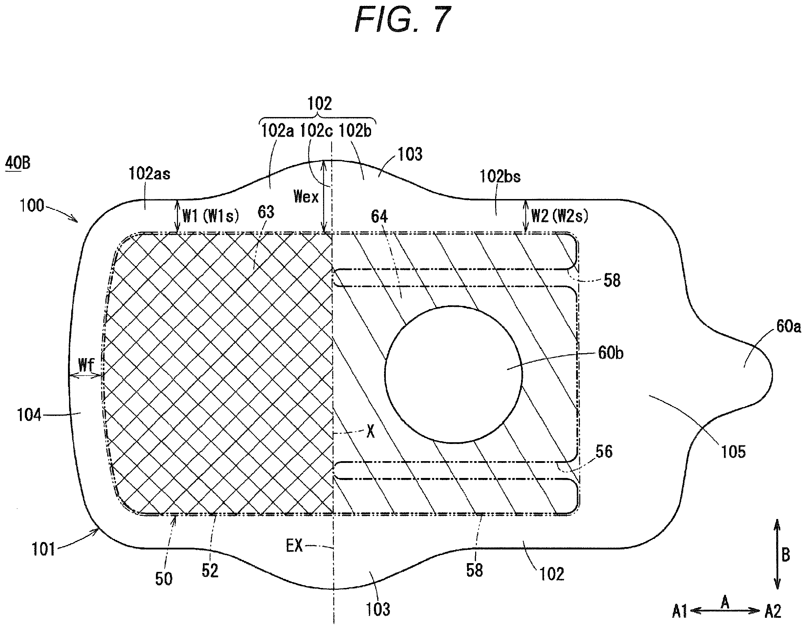

As shown in FIG. 7, a retention structure 40B according to a third embodiment includes a main-body-side attachment sheet 100 having a shape different from a shape the main-body-side attachment sheet 60 described above. A pair of lateral extension parts 102 extending along a direction in which a first section 63 and a second section 64 are arranged is provided as a periphery section 101 on both sides of the main-body-side attachment sheet 100 in a transverse direction. Each of the lateral extension parts 102 has a projection 103 projecting outward in the arrow B directions at a substantially central portion in the arrow A directions of the main-body-side attachment sheet 100.

The projection 103 is formed in an arc shape having a large radius of curvature, and, at a top of the projection 103, an extension line EX obtained by extending a boundary X between the first section 63 and the second section 64 intersects. That is, each of the lateral extension parts 102 includes a boundary adjacent part 102c at a portion corresponding to the top of the projection 103, and a boundary adjacent part 102c projects most in each of the lateral extension parts 102.

A first section adjacent part 102a provided on the arrow A1 side of the boundary adjacent part 102c gradually narrows from the projection 103 toward the direction of the arrow A1. The first section adjacent part 102a has a width W1s narrowest among a portion where an outer edge of the main-body-side attachment sheet 100 extends in parallel to arrow A directions (hereinafter, referred to as a first-section-side straight part 102as). Similarly, a second section adjacent part 102b provided on the arrow A2 side of the boundary adjacent part 102c gradually narrows from the projection 103 toward the direction of the arrow A2. The second section adjacent part 102b has a width W2s narrowest among a portion where an outer edge of the main-body-side attachment sheet 100 extends in parallel to arrow A directions (hereinafter, referred to as a second-section-side straight part 102bs).

The width W1s of the first-section-side straight part 102as and the width W2s of the second-section-side straight part 102bs substantially coincide with each other (Wls = W2s). For example, the widths W1s and W2s are set within a range of about 5 mm to 7 mm. A width Wex of the boundary adjacent part 102c is wider than the width W1s of the first-section-side straight part 102as and the width W2s of the second-section-side straight part 102bs (Wex > Wls, W2s). For example, the width Wex of the boundary adjacent part 102c is set within a range of about 8.5 mm to 14 mm.

The periphery section 101 has a one-end extension part 104 extending with a constant width Wf along the arrow B directions on the arrow A1 side of the first section 63. Both sides of the one-end extension part 104 in the arrow B directions are continuous with a pair of first-section-side straight parts 102as. The width Wf of the one-end extension part 104 and the width W1s of the first-section-side straight parts 102as substantially coincide with each other. It should be noted that an another-end extension part 105 is formed in the same shape as the another-end extension part 68 of the first embodiment.

In the retention structure 40B described above, the width Wex of the boundary adjacent part 102c of the main-body-side attachment sheet 100 is wide, by which force to maintain attachment is increased at the boundary adjacent part 102c. Therefore, when the main body 14 moves around the first section 63, peeling of the main-body-side attachment sheet 100 from a body surface can be reduced. Furthermore, of the main-body-side attachment sheet 100, an area to be attached to the body surface is reduced. Therefore, discomfort of the patient can be reduced.

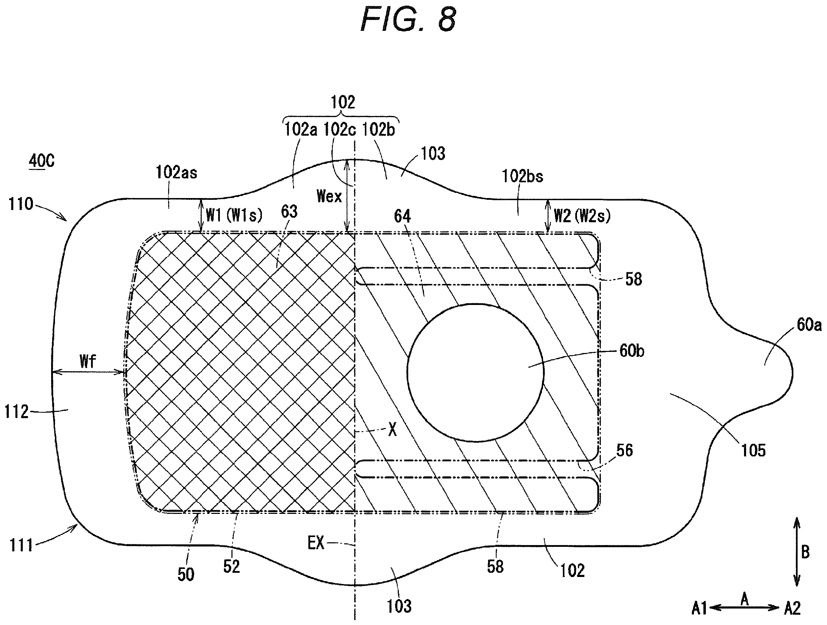

As shown in FIG. 8, a retention structure 40C according to a fourth embodiment includes a main-body-side attachment sheet 110 having a one-end extension part 112 projecting toward the arrow A1 side further than the one-end extension part 104 of the main-body-side attachment sheet 100 in the third embodiment. A width Wf of the one-end extension part 112 is set within a range of, for example, about 8.5 mm to 14 mm, and is set to 10.5 mm in the present embodiment.

In other words, the width Wf of the one-end extension part 112 is set to substantially coincide with a width Wex of a boundary adjacent part 102c or to be slightly wider than the width Wex of the boundary adjacent part 102c. The main-body-side attachment sheet 110 configured as described above has improved followability to a wrinkle generated in a lateral direction of an abdomen, and has more force to maintain attachment of the arrow A1 side of the main body 14, by which peeling of the main-body-side attachment sheet 110 from a body surface can be further reduced.

Technical ideas and effects that may be grasped from the above embodiments will be described below.

One aspect of the present invention is a self-retaining device 10 including a main body 14 and an attachment member (main-body-side attachment sheet 60, 100, 110) that is sheet-shaped and configured to be attached to a body surface of a living body in order to retain the main body 14, in which the attachment member includes a first section 63 fixed to a back surface 22 of the main body 14, a second section 64 that faces the back surface 22 of the main body 14 at a position adjacent to the first section 63 and is able to separate from the main body 14, and a periphery section 65, 101, 111 that is a section adjacent to an outside of the first section 63 and the second section 64 and is configured to be attached to the body surface, the periphery section 65, 101, 111 includes a lateral extension part 66, 102 parallel to a direction in which the first section 63 and the second section 64 are arranged, the lateral extension part 66, 102 has a boundary adjacent part 66c, 102c overlapping an extension line EX of a linear boundary X between the first section 63 and the second section 64, a first section adjacent part 66a, 102a adjacent to the boundary adjacent part 66c, 102c and the first section 63, and a second section adjacent part 66b, 102b adjacent to the boundary adjacent part 66c, 102c and the second section 64, and a width Wex of the boundary adjacent part 66c, 102c is equal to or wider than a maximum width (width W1) of the first section adjacent part 66a, 102a and is wider than a maximum width (width W2) of the second section adjacent part 66b, 102b.

According to the above, in the self-retaining device 10, the width Wex of the boundary adjacent part 66c, 102c is equal to wider than a maximum width of the first section adjacent part 66a, 102a and is equal to or wider than a maximum width of the second section adjacent parts 66b, 102b. Therefore, the boundary adjacent part 66c, 102c is more difficult to peel off from the body surface. That is, an attachment member (main-body-side attachment sheet 60, 100, 110) can increase force to maintain attachment to a body surface in a periphery of the boundary X where a large torque load of vibration of the main body 14 is applied. Therefore, the self-retaining device 10 can stably retain the main body 14 on a body surface of a living body by favorably reducing peeling of an attachment member off from the body surface.

A width Wex of the boundary adjacent part 66c, 102c is set within a range of 1.4 times to 3.5 times a width W2 of second section adjacent parts 66b, 102b. As a result, the self-retaining device 10 can further reduce peeling of the boundary adjacent part 66c, 102c from the body surface.

A difference ΔW obtained by subtracting the width W2 of the second section adjacent parts 66b, 102b from the width Wex of the boundary adjacent part 66c, 102c is set within a range of 2.5 mm to 10 mm. In this case also, the self-retaining device 10 can further reduce peeling of the boundary adjacent part 66c, 102c from the body surface.

The first section 63 includes a sticking part 42 laminated between the main body 14 and an attachment member (main-body-side attachment sheet 60, 100, 110). As a result, in the self-retaining device 10, the first section 63 of the attachment member and the main body 14 are firmly stuck via the sticking part 42, and the main body 14 on a side close to the second section 64 adjacent to the first section 63 can be favorably rise over the body surface.

The attachment member (main-body-side attachment sheet 60, 100, 110) is formed in a shape having a longitudinal direction and a transverse direction, the first section 63 and the second section 64 are arranged in the longitudinal direction of the attachment member, and the boundary adjacent part 66c, 102c is positioned in a middle of three regions obtained by dividing the attachment member into three equal parts along the longitudinal direction. As a result, the self-retaining device 10 can achieve both firm sticking of the main body 14 at the first section 63 and rising of the main body 14 at the second section 64 in a well-balanced manner while reducing peeling of the boundary adjacent part 66c, 102c.

The main body 14 has an operation unit 23 operated by a user, the attachment member (main-body-side attachment sheet 60, 100, 110) has an attachment-member-side exposure hole 60b that exposes the operation unit 23 from a body-surface attachment surface of the attachment member, and the attachment-member-side exposure hole 60b is provided in the second section 64. As a result, it is possible to dispose the first section 63 so as not to overlap the attachment-member-side exposure hole 60b, and the self-retaining device 10 can be operated with the main body 14 being stably fixed and the operation unit 23 rising over the second section 64.

In the attachment member (main-body-side attachment sheet 60), the periphery section 65 has the one-end extension part 67 that is opposite to the second section 64 with respect to the first section 63, is adjacent to the first section 63, and is continuous with the first section adjacent part 66a, and the width W1 of the first section adjacent part 66a and the width Wf of the one-end extension part 67 coincide with the width Wex of the boundary adjacent part 66c. As a result, the attachment member can increase force thereof to maintain attachment in an entire periphery of the first section 63, and can easily follow a wrinkle of an abdomen, the wrinkle being generated in a lateral direction of the attachment member.

Each of the lateral extension parts 102 has, at an intermediate position in an extending direction, a projection 103 that bulges in a direction away from the first section 63 and the second section 64, and the boundary adjacent part 102c is provided at a top of the projection 103. As a result, the attachment member can reduce peeling of the projection 103 from the body surface.

In the attachment member (main-body-side attachment sheet 110), the periphery section 111 has the one-end extension part 112 that is opposite to the second section 64 with respect to the first section 63, is adjacent to the first section 63, and is continuous with the first section adjacent part 102a, and the width Wf of the one-end extension part 112 coincides with the width Wex of the boundary adjacent part 102c or wider than the width Wex of the boundary adjacent part 102c. As a result, the attachment member can reduce peeling of the boundary adjacent part 102c, and can easily follow a wrinkle of an abdomen, the wrinkle being generated in a lateral direction of the attachment member.

The self-retaining device 10 is configured elastically deformable and includes an intermediate member 50 having a plate shape and laminated between the main body 14 and the attachment member (main-body-side attachment sheet 60, 100, 110), in which the intermediate member 50 has a base part 52 overlapping the first section 63 and a first extending part (central piece 56) and second extending part (arm piece 58) that are extending in parallel to each other from the base part 52 and overlapping the second section 64, a first sticking part 70 stuck to the attachment member from the base part 52 to the first extending part is provided on a surface of the intermediate member 50, the surface being on a side close to the attachment member, a second sticking part 80 stuck to the main body 14 from the base part 52 to the second extending part is provided on a surface of the intermediate member 50, the surface being on a side close to the main body 14, the first extending part is not stuck to the main body 14, and the second extending part is not stuck to the attachment member. As a result, the self-retaining device 10 can stably keep the main body 14 and the attachment member stuck to each other even with a configuration in which the main body 14 rises over the second section 64.

In a case where, of the attachment member (main-body-side attachment sheet 60, 100, 110), a side close to the first section 63 including the periphery section 65, 101, 111 is defined as a first region 90 and a side close to the second section 64 including the periphery section 65, 101, 111 is defined as a second region 92 with the boundary X and the extension line EX as the base points, an area of the second region 92 is 1/4 or more of an area of the first region 90. As a result, the self-retaining device 10 can sufficiently ensure the second region 92 of the attachment member and cause the attachment member to follow the body surface.

The second section adjacent parts 66b has a straight part 66b2 parallel to a direction in which the first section 63 and the second section 64 are arranged, and a length Ls of the straight part 66b2 is set within a range of 1 mm to 15 mm. As a result, in the self-retaining device 10, the length of the second section adjacent part 66b can be ensured to some extent, and a portion of the attachment member (main-body-side attachment sheet 60, 100, 110) on the second section 64 side can be stably attached.

## Claims

1. A self-retaining device (10) comprising:
a main body (14); and
an attachment member (22) that is sheet-shaped and configured to be attached to a body surface of a living body in order to retain the main body (14),
wherein the attachment member (22) has
a first section (63) fixed to a back surface (22) of the main body (14),
a second section (64) that faces the back surface (22) of the main body (14) at a position adjacent to the first section (63) and is able to separate from the main body (14), and
a periphery section (65) that is a section adjacent to an outside of the first section (63) and the second section (64) and is configured to be attached to the body surface,
the periphery section (65) includes a lateral extension part (66) parallel to a direction in which the first section (63) and the second section (64) are arranged,
the lateral extension part (66) has a boundary adjacent part (66c) overlapping an extension line of a linear boundary (X) between the first section (63) and the second section (64), a first section adjacent part (66a) adjacent to the boundary adjacent part (66c) and the first section (63), and a second section adjacent part (66b) adjacent to the boundary adjacent part (66c) and the second section (64), and
a width of the boundary adjacent part (66c) is equal to or wider than a maximum width of the first section adjacent part (66a) and is wider than a maximum width of the second section adjacent part (66b).

2. The self-retaining device (10) according to claim 1,
wherein the width of the boundary adjacent part (66c) is set within a range of 1.4 times to 3.5 times a width of the second section adjacent part (66b).

3. The self-retaining device (10) according to claim 1 or 2,
wherein a difference obtained by subtracting the width of the second section adjacent part (66b) from the width of the boundary adjacent part (66c) is set within a range of 2.5 mm to 10 mm.

4. The self-retaining device (10) according to any one of claims 1 to 3,
wherein the first section (63) includes a sticking part laminated between the main body (14) and the attachment member (22).

5. The self-retaining device (10) according to any one of claims 1 to 4,
wherein the attachment member (22) is formed in a shape having a longitudinal direction and a transverse direction,
the first section (63) and the second section (64) are arranged in the longitudinal direction of the attachment member (22), and
the boundary adjacent part (66c) is positioned in a middle of three regions obtained by dividing the attachment member (22) into three equal parts along the longitudinal direction.

6. The self-retaining device (10) according to any one of claims 1 to 5,
wherein the main body (14) has an operation unit (23) operated by a user, the attachment member (22) includes an attachment-member-side exposure hole (60b) that exposes the operation unit (23) from a body-surface attachment surface of the attachment member (22), and
the attachment-member-side exposure hole (60b) is provided in the second section (64).

7. The self-retaining device (10) according to any one of claims 1 to 6,
wherein the periphery section (65) has, in the attachment member (22), a one-end extension part (67, 104, 112) that is opposite to the second section (64) with respect to the first section (63), is adjacent to the first section (63), and is continuous with the first section adjacent part (66a), and
a width of the first section adjacent part (66a) and a width of the one-end extension part (67, 104, 112) coincide with the width of the boundary adjacent part (66c).

8. The self-retaining device (10) according to any one of claims 1 to 6,
wherein the lateral extension part (66) has, at an intermediate position in an extending direction, a projection (103) that bulges in a direction away from the first section (63) and the second section (64), and
the boundary adjacent part (66c) is provided at a top of the projection (103).

9. The self-retaining device (10) according to claim 8,
wherein the periphery section (65) has, in the attachment member (22), a one-end extension part (67, 104, 112) that is opposite to the second section (64) with respect to the first section (63), is adjacent to the first section (63), and is continuous with the first section adjacent part (66a), and
the width of the one-end extension part (67, 104, 112) coincides with the width of the boundary adjacent part (66c) or is wider than the width of the boundary adjacent part (66c).

10. The self-retaining device (10) according to any one of claims 1 to 9, the self-retaining device (10) being configured elastically deformable and further comprising an intermediate member (50) having a plate shape and laminated between the main body (14) and the attachment member (22),
wherein the intermediate member (50) has a base part (52) overlapping the first section (63) and a first extending part and second extending part that are extending in parallel to each other from the base part (52) and overlapping the second section (64),
a first sticking part (70) stuck to the attachment member (22) from the base part (52) to the first extending part is provided on a surface of the intermediate member (50), the surface being on a side close to the attachment member (22),
a second sticking part (80) stuck to the main body (14) from the base part (52) to the second extending part is provided on a surface of the intermediate member (50), the surface being on a side close to the main body (14),
the first extending part is not stuck to the main body (14), and
the second extending part is not stuck to the attachment member (22).

11. The self-retaining device (10) according to any one of claims 1 to 10,
wherein, in a case where, of the attachment member (22), a side close to the first section (63) including the periphery section (65) is defined as a first region (90) and a side close to the second section (64) including the periphery section (65) is defined as a second region (92) with the boundary and the extension line as the base points,
an area of the second region (92) is 1/4 or more of an area of the first region (90).

12. The self-retaining device (10) according to any one of claims 1 to 11,
wherein the second section adjacent part (66b) has a straight part (66b2, 102as, 102bs) parallel to a direction in which the first section (63) and the second section (64) are arranged, and
a length of the straight part (66b2, 102as, 102bs) is set within a range of 1 mm to 15 mm.

## Patentansprüche

1. Selbsthaltende Vorrichtung (10), umfassend:
einen Hauptkörper (14); und
ein Befestigungselement (22), das blattförmig ist und so gestaltet ist, dass es an einer Körperoberfläche eines lebenden Körpers befestigt werden kann, um den Hauptkörper (14) zu halten,
wobei das Befestigungselement (22) aufweist
einen ersten Abschnitt (63), der an einer rückseitigen Oberfläche (22) des Hauptkörpers (14) befestigt ist,
einen zweiten Abschnitt (64), welcher der rückseitigen Oberfläche (22) des Hauptkörpers (14) an einer Position benachbart zu dem ersten Abschnitt (63) zugewandt ist und in der Lage ist, sich von dem Hauptkörper (14) zu trennen, und
einen Umfangsabschnitt (65), bei dem es sich um einen Abschnitt handelt, der benachbart zu einer Außenseite des ersten Abschnitts (63) und des zweiten Abschnitts (64) ist, und der so konfiguriert ist, dass er an der Körperoberfläche befestigt werden kann,
wobei der Umfangsabschnitt (65) ein seitliches Verlängerungsteil (66) parallel zu einer Richtung aufweist, in welcher der erste Abschnitt (63) und der zweite Abschnitt (64) angeordnet sind,
wobei das seitliche Verlängerungsteil (66) eines an die Begrenzung angrenzenden Teils (66c), das eine Verlängerungslinie einer linearen Begrenzung (X) zwischen dem ersten Abschnitt (63) und dem zweiten Abschnitt (64) überlappt, ein an den ersten Abschnitt angrenzendes Teil (66a), das an das an die Begrenzung angrenzende Teil (66c) und den ersten Abschnitt (63) angrenzt, und das an einen zweiten Abschnitt angrenzende Teil (66b), das an das an die Begrenzung angrenzende Teil (66c) und den zweiten Abschnitt (64) angrenzt, aufweist, und
eine Breite des an die Begrenzung angrenzenden Teils (66c) gleich oder breiter als eine maximale Breite des an den ersten Abschnitt angrenzenden Teils (66a) ist und breiter als eine maximale Breite des an den zweiten Abschnitt angrenzenden Teils (66b) ist.

2. Selbsthaltende Vorrichtung (10) nach Anspruch 1,
wobei die Breite des an die Begrenzung angrenzenden Teils (66c) innerhalb eines Bereiches von dem 1,4-fachen bis zu dem 3,5-fachen einer Breite des an den zweiten Abschnitt angrenzenden Teils (66b) festgelegt ist.

3. Selbsthaltende Vorrichtung (10) nach Anspruch 1 oder 2,
wobei eine Differenz, die durch Subtraktion der Breite des an den zweiten Abschnitt angrenzenden Teils (66b) von der Breite des an die Begrenzung angrenzenden Teils (66c) erhalten wird, innerhalb eines Bereiches von 2,5 mm bis 10 mm festgelegt ist.

4. Selbsthaltende Vorrichtung (10) nach einem der Ansprüche 1 bis 3,
wobei der erste Abschnitt (63) ein klebendes Teil umfasst, das zwischen dem Hauptkörper (14) und dem Befestigungselement (22) laminiert ist.

5. Selbsthaltende Vorrichtung (10) nach einem der Ansprüche 1 bis 4,
wobei das Befestigungselement (22) in einer Form ausgebildet ist, die eine Längsrichtung und eine Querrichtung aufweist,
der erste Abschnitt (63) und der zweite Abschnitt (64) in der Längsrichtung des Befestigungselements (22) angeordnet sind, und
das an die Begrenzung angrenzende Teil (66c) in einer Mitte von drei Bereichen angeordnet ist, die durch Unterteilung des Befestigungselements (22) in drei gleiche Teile entlang der Längsrichtung erhalten werden.

6. Selbsthaltende Vorrichtung (10) nach einem der Ansprüche 1 bis 5,
wobei der Hauptkörper (14) eine Betätigungseinheit (23) aufweist, die von einem Benutzer betätigt wird, das Befestigungselement (22) ein Befestigungselement-seitiges Freilegungsloch (60b) aufweist, das die Betätigungseinheit (23) von einer Körperoberfläche-seitigen Befestigungsfläche des Befestigungselements (22) freilegt, und
das Befestigungselement-seitige Freilegungsloch (60b) in dem zweiten Abschnitt (64) vorgesehen ist.

7. Selbsthaltende Vorrichtung (10) nach einem der Ansprüche 1 bis 6,
wobei der Umfangsabschnitt (65) in dem Befestigungselement (22) ein einseitiges Endverlängerungsteil (67, 104, 112) aufweist, das dem zweiten Abschnitt (64) in Bezug auf den ersten Abschnitt (63) gegenüberliegt, an den ersten Abschnitt (63) angrenzt und mit dem an den ersten Abschnitt angrenzenden Teil (66a) durchgehend ist, und
eine Breite des an den ersten Abschnitt angrenzenden Teils (66a) und eine Breite des einseitigen Endverlängerungsteils (67, 104, 112) mit der Breite des an die Begrenzung angrenzenden Teils (66c) übereinstimmen.

8. Selbsthaltende Vorrichtung (10) nach einem der Ansprüche 1 bis 6,
wobei das seitliche Verlängerungsteil (66) an einer Zwischenposition in einer Erstreckungsrichtung einen Vorsprung (103) aufweist, der sich in einer Richtung weg von dem ersten Abschnitt (63) und dem zweiten Abschnitt (64) vorwölbt, und
das an die Begrenzung angrenzende Teil (66c) an einer Oberseite des Vorsprungs (103) vorgesehen ist.

9. Selbsthaltende Vorrichtung (10) nach Anspruch 8,
wobei der Umfangsabschnitt (65) in dem Befestigungselement (22) ein einseitiges Endverlängerungsteil (67, 104, 112) aufweist, das dem zweiten Abschnitt (64) in Bezug auf den ersten Abschnitt (63) gegenüberliegt, an den ersten Abschnitt (63) angrenzt und mit dem an den ersten Abschnitt angrenzenden Teil (66a) durchgehend ist, und
die Breite des einseitigen Endverlängerungsteils (67, 104, 112) mit der Breite des an die Begrenzung angrenzenden Teils (66c) übereinstimmt oder breiter ist als die Breite des an die Begrenzung angrenzenden Teils (66c).

10. Selbsthaltende Vorrichtung (10) nach einem der Ansprüche 1 bis 9, wobei die Selbsthaltevorrichtung (10) elastisch verformbar ausgebildet ist und ferner ein Zwischenelement (50) umfasst, das eine Plattenform aufweist und zwischen dem Hauptkörper (14) und dem Befestigungselement (22) laminiert ist,
wobei das Zwischenelement (50) ein Basisteil (52), das den ersten Abschnitt (63) überlappt, und ein erstes sich erstreckendes Teil und ein zweites sich erstreckendes Teil aufweist, die sich parallel zueinander von dem Basisteil (52) erstrecken und den zweiten Abschnitt (64) überlappen,
ein erstes klebendes Teil (70), das mit dem Befestigungselement (22) von dem Basisteil (52) zu dem ersten sich erstreckenden Teil verklebt ist, auf einer Oberfläche des Zwischenelements (50) vorgesehen ist, wobei die Oberfläche auf einer Seite nahe dem Befestigungselement (22) liegt,
ein zweites klebendes Teil (80), das mit dem Hauptkörper (14) von dem Basisteil (52) zu dem zweiten sich erstreckenden Teil verklebt ist, auf einer Oberfläche des Zwischenelements (50) vorgesehen ist, wobei die Oberfläche auf einer Seite nahe zu dem Hauptkörper (14) liegt,
das erste Verlängerungsteil nicht mit dem Hauptkörper (14) verklebt ist, und
das zweite Verlängerungsteil nicht mit dem Befestigungselement (22) verklebt ist.

11. Selbsthaltende Vorrichtung (10) nach einem der Ansprüche 1 bis 10,
wobei in einem Fall, in dem von dem Befestigungselement (22) eine Seite, die in der Nähe des ersten Abschnitts (63) liegt, einschließlich des Umfangsabschnitts (65) als ein erster Bereich (90) definiert ist und eine Seite, die in der Nähe des zweiten Abschnitts (64) liegt, einschließlich des Umfangsabschnitts (65) als ein zweiter Bereich (92) mit der Begrenzung und der Verlängerungslinie als die Festpunkte definiert sind,
eine Fläche des zweiten Bereichs (92) 1/4 oder mehr einer Fläche des ersten Bereichs (90) beträgt.

12. Selbsthaltende Vorrichtung (10) nach einem der Ansprüche 1 bis 11,
wobei das an den zweiten Abschnitt angrenzende Teil (66b) einen geraden Teil (66b2, 102as, 102bs) parallel zu einer Richtung aufweist, in welcher der erste Abschnitt (63) und der zweite Abschnitt (64) angeordnet sind, und
eine Länge des geraden Teils (66b2, 102as, 102bs) innerhalb eines Bereichs von 1 mm bis 15 mm festgelegt ist.

## Revendications

1. Dispositif autorétentif (10) comprenant :
un corps principal (14) ; et
un élément de fixation (22) qui est en forme de plaque et configuré pour être fixé à une surface de corps d'un corps vivant afin de retenir le corps principal (14),
dans lequel l'élément de fixation (22) a
une première section (63) fixée à une surface arrière (22) du corps principal (14),
une seconde section (64) qui fait face à la surface arrière (22) du corps principal (14) à une position adjacente à la première section (63) et peut se séparer du corps principal (14), et
une section de périphérie (65) qui est une section adjacente à un extérieur de la première section (63) et de la seconde section (64) et est configurée pour être fixée à la surface de corps,
la section de périphérie (65) comporte une partie d'extension latérale (66) parallèle à une direction dans laquelle la première section (63) et la seconde section (64) sont agencées,
la partie d'extension latérale (66) a une partie adjacente de limite (66c) chevauchant une ligne d'extension d'une limite linéaire (X) entre la première section (63) et la seconde section (64), une partie adjacente de première section (66a) adjacente à la partie adjacente de limite (66c) et à la première section (63), et une partie adjacente de seconde section (66b) adjacente à la partie adjacente de limite (66c) et à la seconde section (64), et une largeur de la partie adjacente de limite (66c) est égale à ou plus large qu'une largeur maximale de la partie adjacente de première section (66a) et est plus large qu'une largeur maximale de la partie adjacente de seconde section (66b).

2. Dispositif autorétentif (10) selon la revendication 1, dans lequel la largeur de la partie adjacente de limite (66c) est établie dans une plage de 1,4 fois à 3,5 fois une largeur de la partie adjacente de seconde section (66b).

3. Dispositif autorétentif (10) selon la revendication 1 ou 2,
dans lequel une différence obtenue en soustrayant la largeur de la partie adjacente de seconde section (66b) de la largeur de la partie adjacente de limite (66c) est établie dans une plage de 2,5 mm à 10 mm.

4. Dispositif autorétentif (10) selon l'une quelconque des revendications 1 à 3,
dans lequel la première section (63) comporte une partie collante stratifiée entre le corps principal (14) et l'élément de fixation (22).

5. Dispositif autorétentif (10) selon l'une quelconque des revendications 1 à 4,
dans lequel l'élément de fixation (22) a une forme ayant une direction longitudinale et une direction transversale, la première section (63) et la seconde section (64) sont agencées dans la direction longitudinale de l'élément de fixation (22), et
la partie adjacente de limite (66c) est positionnée à un milieu de trois régions obtenues en divisant l'élément de fixation (22) en trois parties égales le long de la direction longitudinale.

6. Dispositif autorétentif (10) selon l'une quelconque des revendications 1 à 5,
dans lequel le corps principal (14) a une unité d'actionnement (23) actionnée par un utilisateur, l'élément de fixation (22) comporte un trou de mise à nu côté élément de fixation (60b) qui met à nu l'unité d'actionnement (23) depuis une surface de fixation de surface de corps de l'élément de fixation (22), et
le trou de mise à nu côté élément de fixation (60b) est fourni dans la seconde section (64).

7. Dispositif autorétentif (10) selon l'une quelconque des revendications 1 à 6,
dans lequel la section de périphérie (65) a, dans l'élément de fixation (22), une partie d'extension à une extrémité (67, 104, 112) qui est opposée à la seconde section (64) par rapport à la première section (63), est adjacente à la première section (63), et est continue avec la partie adjacente de première section (66a), et
une largeur de la partie adjacente de première section (66a) et une largeur de la partie d'extension à une extrémité (67, 104, 112) coïncident avec la largeur de la partie adjacente de limite (66c).

8. Dispositif autorétentif (10) selon l'une quelconque des revendications 1 à 6,
dans lequel la partie d'extension latérale (66) a, à une position intermédiaire dans une direction d'extension, une saillie (103) qui forme une bosse dans une direction loin de la première section (63) et de la seconde section (64), et
la partie adjacente de limite (66c) est fournie à un sommet de la saillie (103).

9. Dispositif autorétentif (10) selon la revendication 8, dans lequel la section de périphérie (65) a, dans l'élément de fixation (22), une partie d'extension à une extrémité (67, 104, 112) qui est opposée à la seconde section (64) par rapport à la première section (63), est adjacente à la première section (63), et est continue avec la partie adjacente de première section (66a), et
la largeur de la partie d'extension à une extrémité (67, 104, 112) coïncide avec la largeur de la partie adjacente de limite (66c) ou est plus large que la largeur de la partie adjacente de limite (66c).

10. Dispositif autorétentif (10) selon l'une quelconque des revendications 1 à 9, le dispositif autorétentif (10) étant configuré déformable élastiquement et comprenant en outre un élément intermédiaire (50) ayant une forme de plaque et stratifié entre le corps principal (14) et l'élément de fixation (22),
dans lequel l'élément intermédiaire (50) a une partie base (52) chevauchant la première section (63) et une première partie d'extension et une seconde partie d'extension qui s'étendent en parallèle l'une avec l'autre depuis la partie base (52) et chevauchent la seconde section (64),
une première partie collante (70) collée à l'élément de fixation (22) depuis la partie base (52) jusqu'à la première partie d'extension est fournie sur une surface de l'élément intermédiaire (50), la surface étant sur un côté proche de l'élément de fixation (22),
une seconde partie collante (80) collée au corps principal (14) depuis la partie base (52) jusqu'à la seconde partie d'extension est fournie sur une surface de l'élément intermédiaire (50), la surface étant sur un côté proche du corps principal (14),
la première partie d'extension n'est pas collée au corps principal (14), et
la seconde partie d'extension n'est pas collée à l'élément de fixation (22).

11. Dispositif autorétentif (10) selon l'une quelconque des revendications 1 à 10,
dans lequel, dans un cas où, de l'élément de fixation (22), un côté proche de la première section (63) comportant la section de périphérie (65) est défini comme une première région (90) et un côté proche de la seconde section (64) comportant la section de périphérie (65) est défini comme une seconde région (92) avec la limite et la ligne d'extension comme points de base,
une superficie de la seconde région (92) est égale à 1/4 ou plus d'une superficie de la première région (90).

12. Dispositif autorétentif (10) selon l'une quelconque des revendications 1 à 11,
dans lequel la partie adjacente de seconde section (66b) a une partie droite (66b2, 102as, 102bs) parallèle à une direction dans laquelle la première section (63) et la seconde section (64) sont agencées, et
une longueur de la partie droite (66b2, 102as, 102bs) est établie dans une plage de 1 mm à 15 mm.
